# EUROPEAN PATENT APPLICATION

(11) **EP 1 359 108 A1**
(43) Date of publication of application: **05.11.2003**
(21) Application number: 02009748.1
(22) Date of filing: 30.04.2002
(51) Int. Cl.: B65H 63/06

(54) **Method and compact device for detection of defects of yarns during production, in particular chenille yarns**

(71) Applicant: Seltec srl, 50041 Calenzano (IT)
(72) Inventor: Dell' Aglio, Gastone, 50018 Scandicci (FI) (IT); Tosi, Stefano, 50137 Firenze (IT); Baldaccini, Claudio, 50100 Firenze (IT)
(74) Representative: Celestino, Marco

(57) **Abstract**

A method and a device for detection of defects in yarns, in particular but not exclusively chenille yarns, that allows to reduce to the minimum the zones where the yarn cannot be inspected with at least equal definition rate as other existing devices, but with a lower cost of construction. The method can also be actuated directly downstream of the spinning of the yarn, before being spooled on the spindles. The device comprises a body (1) that extends according to a plane and has a through hole (2) that crosses the plane same. The hole is made to let the chenille yarn (10) to pass in the apparatus along a direction crossing the plane. The body has at least four recesses (3) in couples opposite to one another in the central hole (2) and at least a first and a second sender and a first and a second receiver associated each to a respective recess so that each couple sender-receiver is associated to two opposite recesses. A computing unit is provided with an electronic board that extends parallel to the plane along which the body extends, forming a compact system, both with the senders and with the receivers and comprises means for analysing the shadow of the yarn projected onto the receivers from the wave beams coming from the senders. This way, a much easier connection physical and logical is achieved among the components, minimizing the number of interconnections.

## Description

### Field of the invention

The present invention relates to the field of textiles and more precisely it relates to a method for detection of defects on yarns during production. In particular the invention relates to the detection of defects on chenille yarns.

Furthermore, the invention relates to a compact device that carries out this method.

### Background of the invention.

Chenille yarns defects, as well as defects of other yarns having high count, may be classified as long defects or short defects (instantaneous).

Long defects are an increase or a decrease beyond a certain rate of the nominal count of yarn measured on a long portion of yarn.

Short defects, peculiar to chenille, may be classified as "fault" and "burl". The former corresponds to a concentrated decrease of the count of yarn, that in the case of chenille is due to lack of transversal yarn, whereas chenille "burl" defects are due to local thickening of transversal yarns or to transversal yarns of increased length.

A yarn of good quality must be without both long defects and short defects. In particular, if during production defects are detected, the yarn must be cut, the machine must be stopped and the continuity of the yarn must be restored, after having eliminated the defective portion.

Many devices exist, mainly optically controlled, to be mounted on yarn production machines capable of detecting the defects above cited.

These devices use optical sensors that detect the shadow of the yarn projected by a sender of waves when the yarn passes detected by a receiver and processed by a computing unit.

These optical apparatus can comprise a single sender-receiver unit or a first and a second sender-receiver units of wave beams.

In the devices comprising a single sender-receiver unit there is the drawback that often wrong measurements occur, because only a single beam of waves is present. Therefore, it is not possible to measure defects in a direction orthogonal to that of propagation of the waves.

This drawback is surpassed by the optical control devices having a first and a second sender-receiver unit arranged substantially as a cross. The yarn, in fact, slides in the device and is intersected by two orthogonal beams of waves. This way, it is possible to measure defects also in the case of a chenille yarn that has warp yarn segments all on a plane.

However, also in such detection systems wrong signalling can occur, since, the senders and the corresponding receivers are arranged in such a way that wide zones of "shadow" are present, in which it is not possible to make the inspection.

Furthermore, the inspection of yarn defects are not carried out, for chenille, during spinning, but when passing from the spindles to the reels. For this reason also spooled spindles with yarn without defects are controlled with waste of time.

### Summary of the invention

It is an object of the present invention to provide a device for detection of defects in yarns, in particular but not exclusively chenille yarns, which can overcome the problems existing in the prior art above cited.

It is another object of the present invention, to provide a device for detection of defects that allows to reduce to the minimum the zones where the yarn cannot be inspected.

It is a particular object of the device to measure the defects with at least equal definition rate as other existing devices, but with a lower cost of construction.

It is, furthermore, an object of the present invention to provide a method of detection of defects on a yarn that uses the principles of the device according to the above objects.

It is a further object of the present invention to provide a method of detection of defects in chenille yarns and the like that can be actuated directly downstream of the spinning of the yarn, before being spooled on the spindles.

According to a first aspect of the invention a defects detection device on a yarn comprises a body that extends according to a plane and has a through hole that crosses the plane same. The hole is made to let the chenille yarn to pass in the apparatus along a direction crossing the plane.

In particular, the device, according to the present invention, comprises:
- at least four recesses in couples opposite to one another in the central hole;
- at least a first and a second sender and a first and a second receiver associated each to a respective recess so that each couple sender-receiver is associated to two opposite recesses.

Furthermore, the device comprises a computing unit with an electronic board that extends parallel to the plane along which the body extends. Advantageously, the board is connected directly, to form a compact system, both with the senders and with the receivers and comprises means for analysing the shadow of the yarn projected onto the receivers from the wave beams coming from the senders. This way, a much easier connection physical and logical is achieved among the components, minimizing the number of interconnections.

Preferably, the electronic board is operatively connected to means for blocking the movement and for cutting the yarn to coincide with the detection of defects by at least one of the receivers.

Alternatively, the board is connected to a computing unit that stores the type of defect as detected and the defect is associated to a spindle on which the yarn is spooled, whereby it is possible, in a second moment, to eliminate the defects only from the spindles that contain them, processing quickly the spindles with yarn without defects.

To reduce the surface of each receiver and to save costs, the device according to the invention comprises, advantageously, the use of a magnifying lens associated to each receiver for concentrating the image projected from the senders. The embodiment from the invention comprises, furthermore, the use of a prism for conveying the image from a direction parallel to said plane to a direction orthogonal to it at each receiver. This allows to locate the receivers directly on the electronic board and then to obtain substantial advantages both structural and economical.

Preferably, the body has a substantially rectangular shape whereas the through hole has substantially square shape with side faces inclined at 45° with respect to the sides of the body same.

The hole presents, advantageously, recesses that extend for a relevant part of each edge of the square same in order to minimize the shadow zone in which the device cannot carry out the qualitative analysis of the yarn.

Advantageously, the body and the board have a slit for entering the hole to let the passage of the yarn from a position external to the hole to a position in the hole and vice versa.

The electronic board extends parallel to the plane substantially for all the surface of the body, and has a hole corresponding to that of the body.

The method of detection of defects on a yarn comprises the steps of:
- passage of the yarn through two couples of senders-receivers, so that each receiver receives a shadow of the yarn projected from the respective receiver;
- analysis of each shadow detected by each receiver and transmission of a signal of defect in case at least a shadow analysed exceeds from a predetermined threshold value;
and is characterised in that the shadow projected from each sender, according to a first direction, is deviated to a second direction orthogonal to the first direction before reaching the receiver.

The said method comprises, in particular, a magnifying lens associated to each receiver capable of concentrating onto the receivers the beams coming from the senders.

A first embodiment of the method provides the detection of the defects on a yarn directly downstream of the production, before that it is spooled on a spindle. In this case the type of defect detected is stored and is the defect is associated to a spindle on which the yarn is spooled, whereby it is possible in a second moment to eliminate the defects only from the spindles that contain them, processing quickly the spindles with yarn without defects.

Alternatively, the method provides the detection of the defects in a step of "clearing", during the passage of the yarn from the spindles to the reels. In this case, to the detection of a defect a direct cut corresponds of the yarn and the block of its movement upstream and downstream of the cutting.

According to a further aspect of the invention, a method of detection of defects on a yarn comprises the steps of:
- passage of the yarn through at least a couple of senders-receivers, so that each receiver receives a shadow of the yarn projected from the respective receiver;
- analysis of each shadow detected by each receiver and transmission of a signal of defect in case at least a shadow analysed exceeds from a predetermined threshold value,
- and has the characteristic that the detection of said defects is carried out directly downstream of the production, before that the yarn is spooled on a spindle. In this case the type of defect detected is stored and the defect is associated to a spindle on which the yarn is spooled, whereby it is possible in a second moment to eliminate the defects only of the spindles that contain them, processing quickly the spindles with yarn without defects.

### Brief description of the drawings

Further characteristics and advantages of the device of detection of defects in a yarn and of the relative method according to the invention will be made clearer with the following description of an embodiment thereof, exemplifying but not limitative with reference to attached drawings wherein:
- figure 1 shows a perspective view of a device according to the invention of detection of defects on a chenille yarn in operative conditions;
- figure 2 shows top plan view of the device of figure 1;
- figure 3 shows a top plan view of the board that forms the computing unit of the apparatus for detection of defects according to the invention;
- figure 4 shows a cross sectional view of the board of figure 3, according to arrows IV-IV;

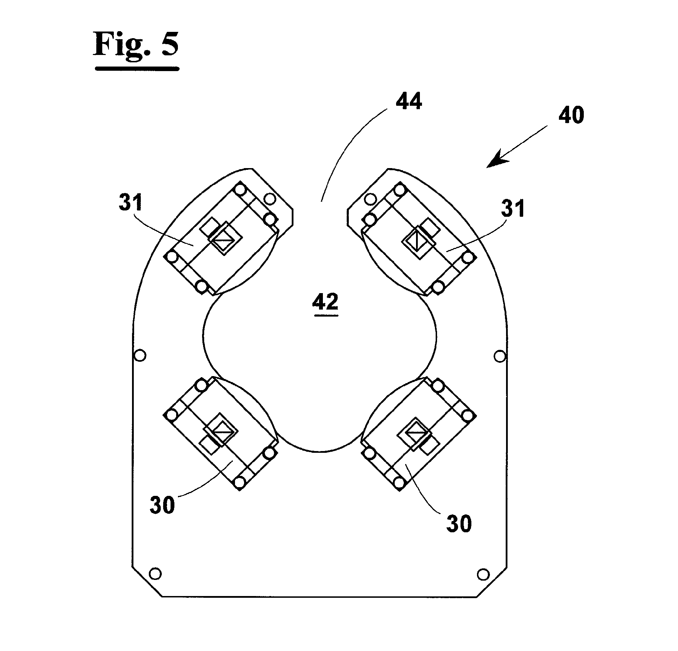

- figure 5 shows in a top plan view the arrangement, on the board of figure 3, of the magnifying lens for concentrating the shadow of the yarn on a sender, or a receiver, according to the invention;

### Description of the preferred embodiment

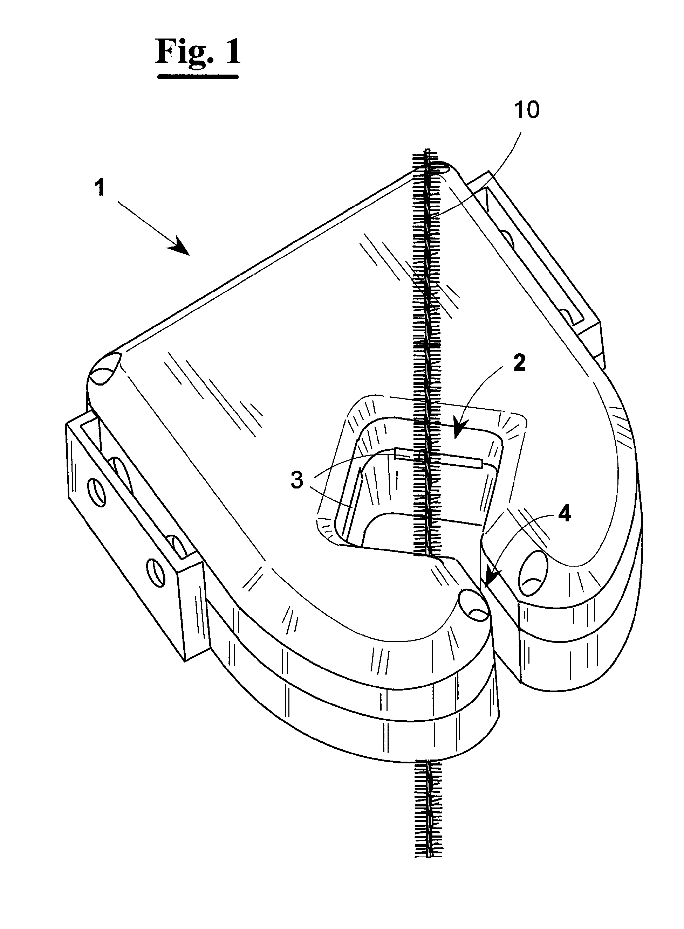

With reference to figure 1, an apparatus for detection of defects on a yarn 10, comprises a body 1 that extends according to a plane and has a hole 2 that crosses it transversally to the plane. In operative conditions the hole 2 is crossed by a yarn 10 and comprises at least four recesses 3, in couples opposite to one another in hole 2.

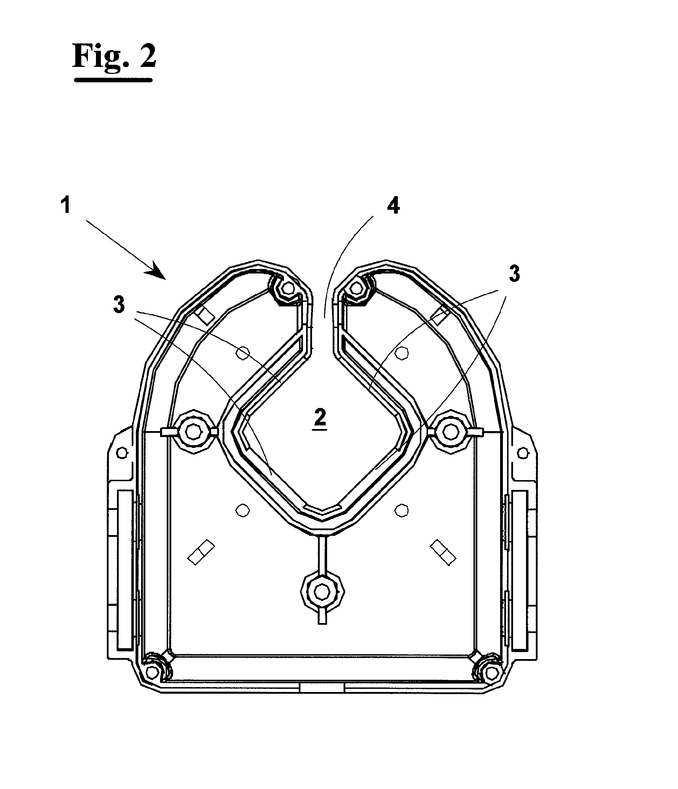

Within each couple of recesses 3, from parts opposite with respect to the hole 2, a sender 21 and a corresponding receiver 22 are housed, of which in figure 2 is shown only the outline.

Preferably, the hole 2 has a substantially square shape and the recesses 3 extend a relevant part of each edge of the square. This allows to minimize the shadow zone in which it is not possible to measure defects of production in the yarn, and then to improve the performance of the device.

To allow the introduction into hole 2 of the yarn 10 without cutting it, and then to start the analysis, the body 1 has an introduction slit 4.

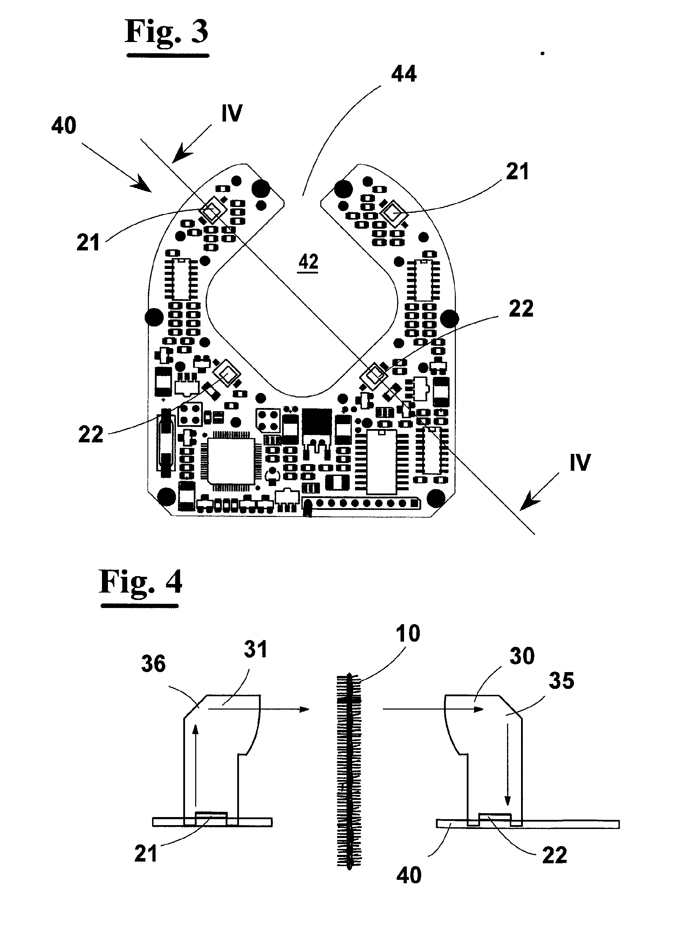

In figure 3 is shown diagrammatically the electronic board 40 that forms the computing unit of the apparatus for detection of defects of yarn 10, according to the invention. This board 40 extends parallel to the plane along which the body 1 extends and is connected directly, in order to form a compact system, to senders 21 and receivers 22. The board 40 has also a slit 44 for yarn 10 and a hole 42, corresponding respectively to slits 4 and to hole 2 of body 1.

The board 40 (figure 3) is made, according to the invention, for being operatively connected to a control unit that operates the means for blocking the movement and for cutting the yarn 10 to coincide with the detection of defects by at least one of the receivers, in case the detection of the defects is carried out during unwinding.

Alternatively, if the step of detection is carried out before winding over spindles, directly on the machines of production of the yarn, the control unit which connects the board stores the defects measured in order to associate the defect to a spindle on which the yarn is spooled, whereby it is possible in a second moment to eliminate the defects only from the spindles that contain them, processing quickly the spindles with yarn without defects.

A particular aspect of the invention relates to the presence of a magnifying lens 30 associated to each receiver 22 capable of concentrating onto the receivers same the image projected from the respective senders 21, and that allows, therefore, to reduce the surface of the receivers 22 same.

To magnifying lens 30 a prism 35 is associated (figure 4) that conveys the image from a direction parallel to the plane to a direction to it orthogonal, and allows to position the receivers 22 directly on the electronic board 40, remarkably making easier the process of production of the apparatus. The same can be made for sender 21, always as shown in figure 4, with respective prism 36 and element optical 31.

The sensor according to the invention is particularly suitable to control defects of chenille yarns directly in the phase of "spinning", as well as when passing from the spindles to the reels. In the first case, the spindles without defects are identified and not subject to control, with considerable save of time.

The foregoing description of a specific embodiment will so fully reveal the invention according to the conceptual point of view, so that others, by applying current knowledge, will be able to modify and/or adapt for various applications such an embodiment without further research and without parting from the invention, and it is therefore to be understood that such adaptations and modifications will have to be considered as equivalent to the specific embodiment. The means and the materials to realise the different functions described herein could have a different nature without, for this reason, departing from the field of the invention. It is to be understood that the phraseology or terminology employed herein is for the purpose of description and not of limitation.

## Claims

1. Defects detection device on a yarn comprising a body that extends according to a plane and having a through hole that crosses said plane, in said hole said yarn passing in a direction crossing said plane, comprising:
- at least four recesses in couples opposite to one another in said hole;
- at least a first and a second sender and a first and a second receiver associated each to a respective recess so that each couple sender-receiver is associated to two opposite recesses;
**characterised in that** it comprises a computing unit with an electronic board that extends parallel to said plane, said board being connected directly, to form a compact system, with said senders and said receivers and it comprises means for analysing the shadow of said yarn projected on said receivers from the wave beams coming from said senders.

2. Device according to claim 1, wherein said board is operatively connected to means for blocking the movement and for cutting said yarn to coincide with the detection of defects by at least one of said receivers.

3. Device according to claim 1, wherein said board is connected to a computing unit that stores the type of defect detected and associates said defect to a spindle on which said yarn is spooled, whereby it is possible, in a second moment, to eliminate the defects only from the spindles that contain them, processing quickly the spindles with yarn without said defects.

4. Device according to claim 1, wherein said board extends parallel to said plane substantially for all the surface of said body.

5. Device according to claim 1, wherein said body and said board have a slit for entering said hole to let the passage of the yarn from a position external to the hole to a position in the hole and vice versa.

6. Device according to claim 1, wherein each receiver is associated to a magnifying lens for concentrating on said receivers the image projected from said senders, and to a prism for conveying said image from a direction parallel to said plane to a direction orthogonal to said plane.

7. Device according to claim 1, wherein said hole has substantially square shape, said recesses extending for a relevant part of each edge of said square.

8. Device according to claim 1, wherein said body has a substantially rectangular shape and said hole has a substantially square shape, the sides of said hole being inclined at 45° with respect to the sides of said body.

9. Method of detection of defects on a yarn comprising the steps of:
- passage of said yarn through two couples of senders-receivers, so that each receiver receives a shadow of said yarn projected from the respective receiver;
- analysis of each shadow detected by each receiver and transmission of a signal of block in case at least a shadow analysis exceeds from a predetermined threshold value;
**characterised in that** the shadow projected from each sender, according to a first direction, is deviated to a second direction orthogonal to said first direction before reaching said receiver.

10. Method of detection of defects on a yarn, according to claim 9, wherein said deviation of direction of said shadow is made after having concentrated on said receivers said beams coming from said senders by means of a magnifying lens associated to each receiver.

11. Method of detection of defects on a yarn, according to claim 9, wherein the detection of the defects on a yarn is made directly downstream of the production, before that it is spooled on a spindle, in order to store the type of defect detected and of associate said defect to a spindle on which said yarn is spooled, whereby it is possible in a second moment to eliminate the defects only of the spindles that contain them, processing quickly the spindles with said yarn without said defects or with a number of said defects lower than a predetermined amount.

12. Method of detection of defects on a yarn, according to claim 9, wherein the detection of the defects is carried out in a step of "clearing", at the passage of said yarn from the spindles of production to the reels, so that to the detection of a defect the direct cut of the yarn and the block of its movement upstream of and downstream of the cutting corresponds.

13. Method of detection of defects on a yarn comprising the steps of:
- passage of said yarn through at least a couple of senders-receivers, so that each receiver receives a shadow of said yarn projected from the respective receiver;
- analysis of each shadow detected by each receiver and transmission of a signal of block in case at least a shadow analysis exceeds from a predetermined threshold value;
**characterised in that** the detection of the defects on said yarn is made directly downstream of the production, before that it is spooled on a spindle.

14. Method of detection of defects on a yarn, according to claim 13, comprising the further steps of:
- storing the type of defect detected,
- associating said defect to a spindle on which said yarn is spooled;
- eliminating in a second moment the defects only of the spindles that contain them, processing quickly the spindles without said defects or with a number of said defects lower than a predetermined amount.
